(19) Europäisches Patentamt
European Patent Office
Office européen des brevets

(11) Numéro de publication: **0 252 815 B1**

(12) **FASCICULE DE BREVET EUROPEEN**

(45) Date de publication du fascicule du brevet: **11.07.90**

(51) Int. Cl.$^5$: **C 07 C 69/96,** C 07 C 68/06

(21) Numéro de dépôt: **87401537.3**

(22) Date de dépôt: **02.07.87**

(54) **Procédé de fabrication des carbonates de bromo-1 alkyle et d'hydrocarbyle.**

(30) Priorité: **11.07.86 FR 8610185**

(43) Date de publication de la demande:
**13.01.88 Bulletin 88/02**

(45) Mention de la délivrance du brevet:
**11.07.90 Bulletin 90/28**

(84) Etats contractants désignés:
**CH DE GB IT LI SE**

(56) Documents cités:
**EP-A-0 108 547**
**GB-A-2 123 821**

(73) Titulaire: **SOCIETE NATIONALE DES POUDRES ET EXPLOSIFS**
**12, quai Henri IV**
**F-75181 Paris Cédex 04 (FR)**

(72) Inventeur: **Wooden, Gary**
**Bâtiment 7C Résidence de Bel Air**
**F-91540 Mennecy (FR)**
Inventeur: **Sennyey, Gérard**
**1, place des 4 Pavés**
**F-91190 Gif sur Yvette (FR)**
Inventeur: **Senet, Jean-Pierre**
**79, rue de la Gare Herbeauvilliers-Buthiers**
**F-77760 La Chapelle La Reine (FR)**

(74) Mandataire: **Pech, Bernard**
**SNPE - Service Propriété Industrielle 12, Quai Henri IV**
**F-75181 Paris Cédex 04 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 252 815 B1

**Description**

L'invention concerne un nouveau procédé de préparation des carbonates de bromo-1 alkyle et d'hydrocarbyle. L'invention concerne également de nouveaux carbonates de bromo-1 alkyle et d'hydrocarbyle.

Très peu de carbonates alpha-bromés sont connus car jusqu'à ces dernières années leur préparation était restée difficile.

Il est possible de préparer le carbonate de bromo-1 éthyle et d'éthyle par réaction d'un halogénoformiate de bromo-1 éthyle sur l'éthanol selon le schéma:

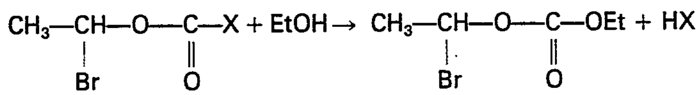

X représentant le chlore ou le brome.

La difficulté de ce procédé réside dans l'obtention de l'halogénoformiate de départ:

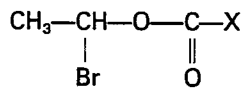

Selon la demande de brevet européen EP—108 547 on effectue une bromation radicalaire du chloroformiate d'éthyle ou du bromoformiate d'éthyle.

Cette bromation radicalaire présente des inconvénients. Les lampes UV employées de préférence aux initiateurs de radicaux libres sont des grands consommateurs d'énergie et cela d'autant plus que les durées de réaction par cette méthode sont importantes. On obtient toujours des sous-produits bromés variés.

Il est également possible de préparer le bromoformiate de bromo-1 éthyle par réaction de l'acétaldéhyde sur le bromophosgène comme décrit dans la demande de brevet français FR—2 532 933. Mais il est alors nécessaire d'utiliser des catalyseurs et le bromophosgène n'est pas un réactif commercialisé. Il est peu stable et très difficile à préparer. On le fabrique par exemple par réaction de l'oxyde de carbone avec le brome mais cette réaction nécessite des installations industrielles très spécifiques.

Un autre procédé de préparation du carbonate de bromo-1 éthyle et d'éthyle consiste à bromer de façon radicalaire le carbonate de diéthyle. On retrouve les mêmes inconvénients des méthodes radicalaires décrits précédemment: obtention de sous-produits, forte demande énergétique. En outre ce procédé ne peut convenir à l'obtention d'un certain nombre d'autres carbonates de bromo-1 éthyle car selon la structure du carbonate de départ le brome se fixera en plus grande quantité sur le groupe hydrocarboné autre que le groupe éthyle, par exemple lorsque l'on essaie de fixer le brome sur le carbonate d'éthyle et d'isopropyle on obtient de façon prépondérante le carbonate d'éthyle et de bromo-1 isopropyle.

On peut également utiliser le carbonate de chloro-1 éthyle et d'éthyle comme matière de départ et effectuer une substitution du chlore par le brome au moyen d'un sel de brome en grand excès tel que le bromure de lithium ou d'ammonium comme indiqué dans la demande EP No. 108 547. Cette méthode présente également des inconvénients car du fait qu'il s'agit d'un équilibre la réaction est incomplète.

On obtient par exemple avec un excès de 300% de bromure de lithium non pas le produit pur mais un mélange du carbonate bromé et de 35% de carbonate de chloro-1 éthyle et d'éthyle comme mentionné dans l'exemple 12 de cette demande et la séparation du carbonate alpha-chloré et du carbonate alpha-bromé est très difficile comme pour tous les autres carbonates d'alkyle inférieurs.

Dans la demande de brevet GB No. 2 123 821 il est décrit un procédé dans lequel le carbonate de chloro-1 éthyle et d'éthyle est également mis à réagir avec un bromure alcalin. La réaction est effectuée dans un système solvant à deux phases comprenant une phase aqueuse et une phase organique en présence d'un catalyseur de transfert de phase mais il est nécessaire d'utiliser également un très grand excès de bromure alcalin et une grande quantité de solvant. La réaction est longue et nécessite plusieurs opérations. La récupération du carbonate alpha-bromé n'est pas aisée. Les solvants doivent être débarassés des produits secondaires pour éviter une pollution.

Très récemment un nouveau procédé de préparation de carbonates de bromo-1 éthyle et d'hydrocarbyle a été proposé dans la demande FR No. 2 573 756. Selon ce procédé on ajoute de l'acide bromohydrique sur un carbonate de vinyle et d'hydrocarbyle mais ce procédé ne permet pas d'obtenir des carbonates alpha-bromés autres que ceux de bromo-1 éthyle et le chloroformiate de vinyle utile pour préparer le carbonate de départ n'est pas très facile à obtenir.

Il existait par conséquent un besoin d'un nouveau procédé pour préparer une grande variété de carbonates de bromo-1 alkyle et d'hydrocarbyle que ne présente pas les inconvénients précédemment décrits.

L'objet de l'invention est précisemment de proposer un tel procédé.

Selon ce nouveau procédé on prépare les carbonates de bromo-1 alkyle et d'hydrocarbyle de formule:

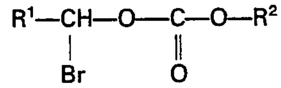

dans laquelle $R^1$ représente un radical aliphatique linéaire ou ramifié en $C_1$ à $C_{20}$, ou cycloaliphatique en $C_5$ à $C_{15}$, et $R^2$ représente un radical substitué ou non, aliphatique en $C_1$ à $C_{12}$, cycloaliphatique en $C_5$ à $C_{20}$ ou aromatique en $C_6$ à $C_{14}$, par réaction d'un carbonate alpha-chloré de formule

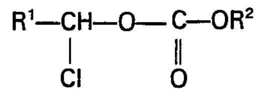

dans laquelle $R^1$ et $R^2$ ont les significations précédentes avec un composé anhydre de formule ZBr dans laquelle Z représente un atome d'hydrogène, le groupe

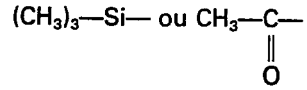

et peut également représenter un atome de brome lorsque $R^2$ est différent du radical aromatique en présence d'un catalyseur choisi parmi les amines hétéroaromatiques, les halogénures d'ammonium ou de phosphonium quaternaires, les halogénures de guanidinium hexasubstitués, les halogénures de métaux alcalins associés à un complexant de leur cation ou les halogénures de métaux alcalino-terreux, en quantité comprise entre 0,5 et 10% en mole par rapport au carbonate alphachloré et à une température comprise entre 40°C et 150°C.

Le schéma réactionnel est le suivant:

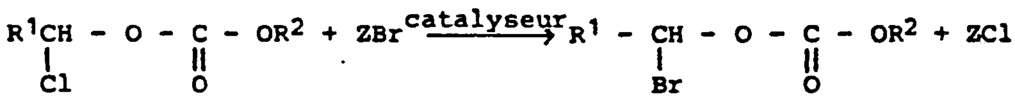


Les carbonates alpha-chlorés utilisés comme matière de départ se trouvent dans le commerce ou peuvent être préparés par des méthodes connues, par exemple par réaction des chloroformiates alpha-chlorés sur les alcools ou les phénols comme indiqué dans les demandes de brevets FR No. 2 559 764 et EP No. 185578.

Le radical $R^1$ est de préférence un radical aliphatique en $C_1$ à $C_8$ ou cycloaliphatique en $C_5$ à $C_{10}$.

$R^2$ est de préférence un radical substitué ou non, aliphatique en $C_1$ à $C_8$, cycloaliphatique en $C_5$ à $C_{10}$ ou aromatique en $C_6$ à $C_{10}$.

Le ou les substituants de $R^2$ sont choisis dans le groupe qui comprend des radicaux aryle, aryloxy, alkoxy, les atomes de chlore, fluor et brome.

Le composé ZBr doit être anhydre. Généralement on l'utilise en quantité comprise entre 1 et 2 moles par mole de carbonate alphachloré, de préférence entre 1,1 et 1,5 moles.

De préférence on utilise comme composé ZBr, HBr,

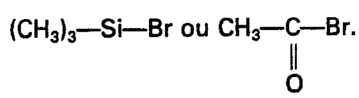

Un catalyseur est nécessaire pour obtenir de bons rendements.

Comme exemple de catalyseurs on peut citer la pyridine, la diméthylamino-4 pyridine, les chlorures et bromures d'ammonium ou de phosphonium quaternaires, le chlorure d'hexabutylguanidinium, le chlorure de potassium associé au 18-couronne-6, le bromure de magnésium.

Les quantités de catalyseurs mises en oeuvre sont de préférence comprises entre 1 et 5% en mole par rapport au carbonate alphachloré.

Le mélange réactionnel est généralement chauffé et de préférence à une température comprise entre 60 et 120°C.

La réaction peut être réalisée avec ou sans solvant. Comme solvants qui conviennent bien on peut citer les solvants aromatiques comme le toluène et les solvants aliphatiques comme le bromo-2 butane ou l'acétate d'éthyle.

Le procédé de l'invention est simple. Sa mise en oeuvre ne demande ni installations couteuses, ni une importante dépense d'énergie. Les matières premières sont facilement disponibles. Il permet d'obtenir de nombreux carbonates alpha-bromés avec une bonne pureté et de bons rendements et en particulier des carbonates alpha-bromés qui n'avaient jamais pu être préparés auparavant.

Les carbonates de bromo-1 alkyle et d'hydrocarbyle préparés selon le procédé de l'invention sont des intermédiaires de synthèse qui peuvent, par exemple, avantageusement remplacer dans de nombreuses réactions leurs homologues chlorés moins réactifs ou iodés plus instables.

Une de leurs applications importantes est la modification des fonctions acide carboxylique avec introduction du groupe carbonate, dans divers composés. La réaction se schématise comme suit:

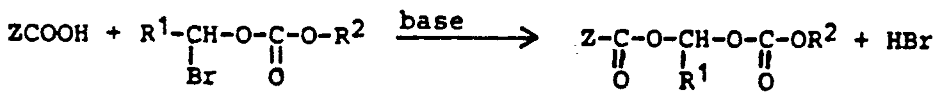


Cette application est notamment décrite dans les brevets US No. 4 426 391 et dans les demandes de brevet EP No. 54 512, EP No. 108 547, FR No. 2 532 933 et 2 387 988.

Ainsi, selon l'enseignement de la demande de brevet français No. 2 532 933 les carbonates alpha-bromés trouvent une application préférentielle comme agents de blocage de certains antibiotiques comme la bac-ampicilline.

Ils peuvent également réagir sur les dérivés de l'acide phosphorique comme indiqué dans la demande de brevet DE No. 2 628 410:

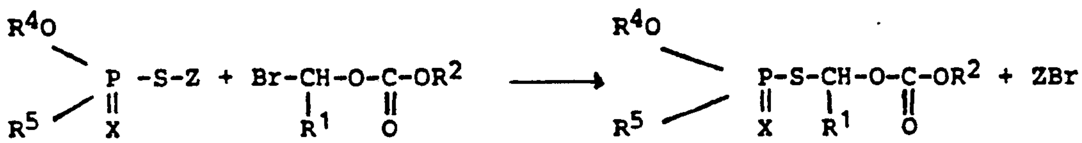

Les composés obtenus selon ce schéma réactionnel trouvent une application intéressante comme produits insecticides.

Comme la liaison C—Br est beaucoup plus labile que la liaison C—Cl, on pourra utiliser les carbonates alpha-bromés en quantité inférieure aux carbonates alpha-chlorés correspondants. Les conditions réactionnelles seront plus douces et de ce fait on évitera une certaine dégradation du composé de départ. Les rendements seront plus élevés.

Les carbonates alpha-bromés sont d'autre part beaucoup plus stables que les carbonates iodés correspondants ce qui permettra lorsqu'ils les remplaceront d'obtenir des produits de pureté plus élevée et en quantité supérieure.

Les exemples qui suivent illustrent l'invention sans toutefois la limiter.

## Exemple 1

Préparation du carbonate de bromo-1 éthyle et d'isopropyle

Dans un réacteur muni d'un agitateur, d'un thermomètre, d'un réfrigérant et d'un tube d'introduction de gaz, on introduit 13,11 g (78,7 mmol) de carbonate d'alpha-chloroéthyle et d'isopropyle et 0,35 g (1,09 mmol; 0,014 eq.) de bromure de tétra n-butyl ammonium. On chauffe le mélange à 80°C et fair passer en 6 heures un courant gazeux de 38 g d'acide bromhydrique anhydre.

On dégaze le milieu réactionnel à l'argon et on distille sous pression réduite.

On recueille ainsi 13,63 g (rendement: Rdt = 82%) du carbonate attendu de caractéristiques:

Ebullition 80—83°C/18 mmHg

IR ν (C=O): 1760 $cm^{-1}$

RMN $^1$H ($CDCl_3$, δ ppm): 6,60 (q, J=6Hz, 1H); 4,91 (sept, J=6Hz, 1H); 2,00 (d, J=6Hz, 3H); 1,3 (d, J=6Hz, 6H).

## Exemple 2

Préparation du carbonate de bromo-1 éthyle et d'éthyle

On prépare le catalyseur, $MgBr_2\cdot(Et_2O)_x$ à partir de 0,15 g (0,006 mol) de magnésium et 1,18 g (0,006 mol) de dibromo-1,2 éthane dans 10 ml d'éther. Après consommation du magnésium, on ajoute la phase inférieure à 20,2 g (0,13 mol) de carbonate de chlore-1 éthyle et d'éthyle. On chauffe le mélange à 60—65°C et on introduit lentement, en 5 h, 36,6 g (0,23 mol) de brome.

Après 20 h d'agitation à 60°C, on laisse refroidir, on ajoute 25 ml de $CH_2Cl_2$, on lave à l'eau puis avec une solution aqueuse de $Na_2S_2O_3$.

On sèche sur $MgSO_4$, on filtre, on concentre, on distille sous pression réduite et on recueille 11,2 g (rendement 43%) du carbonate attendu.

Ebullition 62—66°C/18 mm Hg

IR ν (C=O): 1760 $cm^{-1}$

RMN$^1$H ($CDCl_3$, δ ppm): 6,58 (q, J=6Hz, 1H); 4,20 (q, J=7Hz, 2H); 1,97 (d, J=6Hz, 3H); 1,30 (t, J=7Hz, 3H).

## Exemples 3 et 4

Préparation du carbonate de bromo-1 éthyle et d'éthyle et du carbonate de bromo-1 pentyle et de méthyle

On opére comme à l'exemple 1. Les réactifs utilisés et les résultats obtenus sont indiqués dans le tableau ci-dessous.

| EX. | Cl<br>\|<br>RCHOCOR'<br>‖<br>O | Catalyseur | HBr | T°C | Durée | Rdt |
|---|---|---|---|---|---|---|
| 3 | Cl<br>\|<br>$CH_3CHOCOCH_2CH_3$<br>‖<br>O<br>37,4g;0,245mol | (b)<br>$Mg\ Br_2.(Et_2O)_x$<br>0,0093mol | 35,1 g;<br>0,434 mol | 70°C | 24 h | 76% |
| 4 | Cl O<br>\| ‖<br>$C_4H_9CHOCOCH_3$<br>30,5g;0,169mol | 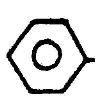$-CH_2NBu_3^{\oplus}\ Cl^{\ominus}$<br>1,0 g;0,003mol | 19,4 g;<br>0,24 mol | 80-<br>85°C | 7h | (a)<br>34% |

a = rendement non optimisé

b = $MgBr_2.(Et_2O)_x$ préparé à partir de Mg + $BrCH_2CH_2Br$ dans l'éther comme à l'exemple 2.

## Exemple 5

Préparation du carbonate de bromo-1 éthyle et de phényle

Dans un appareil de distillation muni d'une colonne Vigreux de 15 cm, d'un thermomètre, et d'un agitateur, on introduit 15,4 g (0,077 mol) de carbonate de chloro-1 éthyle et de phényle, 0,44 g (0,0014 mol) de bromure de tétrabutylammonium et 13,7 g (0,090 mol) de bromure de triméthylsilane. On chauffe le mélange à 90°C pendant 24 h et on élimine le chlorure de triméthylsilane libéré par distillation. Puis, le produit attendu est distillé. On en obtient 17,2 g (Rdt = 91%).

Ebullition 72—77°C/0,04 mm Hg.

IR ν (C=O): 1775 $cm^{-1}$

RMN$^1$H ($CDCl_3$, δ ppm): 7,6—6,9 (m, 5H); 6,56 (q, J=6Hz, 1H); 1,98 (d, J=6Hz, 3H).

## Exemples 6 à 8

Préparation du carbonate de bromo-1 éthyle et d'éthyle

On opère comme à l'exemple 5 à partir du carbonate de chloro-1 éthyle et d'éthyle en présence de différents catalyseurs et en remplaçant le bromure de triméthylsilane par le bromure d'acétyle. Les quantités de réactifs utilisées et les résultats obtenus sont indiqués dans le tableau ci-dessous.

| EX | 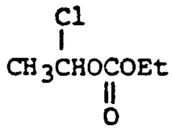 | Catalyseur | 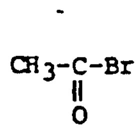 | T°C | Durée | 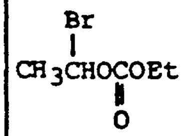<br>Rendement |
|---|---|---|---|---|---|---|
| 6 | 20,5g;<br>0,13 mol | $(C_8H_{17})_3\overset{\oplus}{N}Me\overset{\ominus}{Cl}$<br>0,67 g;<br>0,0017 mol | 19,7 g;<br>0,16 mol | 100-<br>105 | 26 h | 73 % |
| 7 | 20,1 g;<br>0,13 mol | 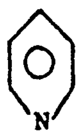<br>0,50 g;0,006 mol | 21,6 g;<br>0,18 mol | 95 | 24 h | 63 % |
| 8 | 20,1g;<br>0,13 mol | KCl - 18-C-6<br>0,80g;0,011 mol<br>1,20g;0,0045 mol | 20,4 g;<br>0,17 mol | 95 | 24 h | 55 % |

Exemple 9

Préparation du carbonate de bromo-1 éthyle et d'isopropyle

Dans un appareil muni d'un thermomètre, d'un agitateur, d'un soxhlet rempli de $CaCl_2$ et surmonté d'un réfrigérant à carboglace et acétate d'éthyle (pour recycler l'excès de HBr) et d'un tube d'introduction de gaz, on introduit 35,2 g (0,21 mol) de carbonate de chloro-1 éthyle et d'isopropyle, 0,71 g (0,0022 mol) de bromure de tétrabutylammonium, et 40 ml de bromo-2 butane.

On chauffe le mélange à 95—104°C et on fait passer en 8 heures un courant gazeux de 26,1 g (0,32 mol) d'acide bromhydrique anhydre.

On dégaze le milieu réactionnel à l'argon, on élimine le bromo-2 butane, on distille sous pression réduite et on obtient 40 g du produit attendu (Rdt = 90%).

Ebullition 82°/18 mmHg.

Exemple 10

Préparation du carbonate de bromo-1 éthyle et d'isopropyle

On chauffe une solution de carbonate de chloro-1 éthyle et d'isopropyle (16,7 g, 0,10 mol) et de chlorure d'hexabutyl guanidinium (0,43 g, 0,0011 mol) dans le toluène (40 ml) à 115° et on fait passer pendant 7 heures un courant gazeux de 15 g (0,186 mol) d'acide bromhydrique anhydre.

On dégaze le milieu réactionnel à l'argon, on élimine le toluène, on distille le produit sous pression réduite et on obtient 16,0 g du produit attendu (Rdt = 76%).

**Revendications**

1. Procédé de préparation des carbonates de bromo-1 alkyle de formule

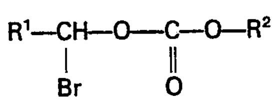

dans laquelle $R^1$ représente un radical aliphatique linéaire ou ramifié en $C_1$ à $C_{20}$ ou cycloaliphatique en $C_5$ à $C_{15}$, et $R^2$ représente un radical aliphatique en $C_1$ à $C_{12}$, cycloaliphatique en $C_5$ à $C_{20}$ ou aromatique en $C_6$ à $C_{14}$, le radical $R^2$ pouvant porter un ou plusieurs substituants choisis dans le groupe qui comprend les radicaux aryle, aryloxy, alkoxy, les atomes de chlore, fluor et brome, caractérisé en ce que l'on fait réagir un carbonate alpha-chloré de formule

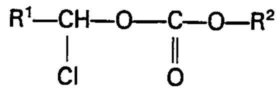

dans laquelle $R^1$ et $R^2$ ont les significations précédentes avec un composé anhydre de formule ZBr dans laquelle Z représente un atome d'hydrogène, le groupe

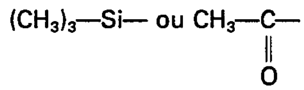

et lorsque $R^2$ est différent du radical aromatique représente également un atome de brome, en présence d'un catalyseur choisi parmi les amines hétéroaromatiques, les halogénures d'ammonium ou de phosphonium quaternaires, les halogénures de guanidinium hexasubstitués, les halogénures de métaux alcalins associés à un complexant de leur cation ou les halogénures de métaux alcalino-terreux, en quantité comprise entre 0,5 et 10% en mole par rapport au carbonate alpha-chloré et à une température comprise entre 40°C et 150°C.

2. Procédé selon la revendication 1 caractérisé en ce que $R^1$ représente un radical aliphatique en $C_1$ à $C_8$ ou cycloaliphatique en $C_5$ à $C_{10}$.

3. Procédé selon la revendication 1 ou 2 caractérisé en ce que $R^2$ représente un radical aliphatique en $C_1$ à $C_8$, cycloaliphatique en $C_5$ à $C_{10}$ ou aromatique en $C_6$ à $C_{10}$, le radical $R^2$ pouvent porter un ou plusieurs substituants choisis dans le groupe qui comprend les radicaux aryle, aryloxy, alkoxy, les atomes de chlore, fluor et brome.

4. Procédé selon l'une quelconque des revendications précédentes caractérisé en ce que le composé ZBr est utilisé en quantité comprise entre 1,05 et 2 moles par mole de carbonate alpha-chloré, de préférence entre 1,1 et 1,5.

5. Procédé selon l'une quelconque des revendications précédentes, caractérisé en ce que le composé ZBr utilisé est HBr,

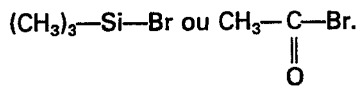

6. Procédé selon l'une quelconque des revendications précédentes, caractérisé en ce que le catalyseur est utilisé en quantité compris entre 1 et 5% en mole par rapport au carbonate alpha-chloré.

7. Procédé selon l'une quelconque des revendications précédentes, caractérisé en ce que le catalyseur est choisi parmi la pyridine, la diméthylamino-4 pyridine, les chlorures et bromures d'ammonium ou de phosphonium quaternaires, le chlorure d'hexabutylguanidinium, le chlorure de potassium associé au 18-couronne-6, le bromure de magnésium.

8. Procédé selon l'une quelconque des revendications précédentes, caractérisé en ce que la température de la réaction est comprise entre 60° et 120°C.

9. Procédé selon l'une quelconque des revendications précédentes, caractérisé en ce que la réaction a lieu en présence d'un solvant.

10. Procédé selon la revendication précédente caractérisé en ce que le solvant est choisi dans le groupe qui comprend les solvants aromatiques et les solvants aliphatiques.

**Patentansprüche**

1. Verfahren zur Herstellung von 1-Bromalkylcarbonaten der Formel

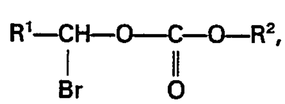

in der bedeuten:

$R^1$ eine geradkettige oder verzweigte aliphatische $C_{1-20}$-Gruppe oder eine cycloaliphatische $C_{5-15}$-Gruppe und

$R^2$ eine aliphatische $C_{1-12}$-Gruppe, eine cycloaliphatische $C_{5-20}$-Gruppe oder eine aromatische $C_{6-14}$-Gruppe, wobei der Substituent $R^2$ einen oder mehrere unter Aryl, Aryloxy, Alkoxy, Chlor, Fluor und Brom ausgewählte Substituenten aufweisen kann, gekennzeichnet durch Umsetzung eines alpha-chlorierten Carbonats der Formel

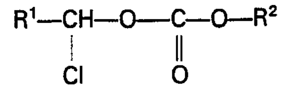

mit $R^1$ und $R^2$ wie oben mit einer wasserfreien Verbindung der Formel ZBr, in der Z ein Wasserstoffatom, die Gruppe

$(CH_3)_3$—Si— oder 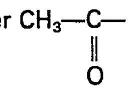

sowie, wenn $R^2$ keine aromatische Gruppe ist, ebenfalls ein Bromatom bedeutet, in Gegenwart eines unter den heteroaromatischen Aminen, quaternären Ammoniumhalogeniden, quaternären Phosphoniumhalogeniden, hexasubstituierten Guanidiniumhalogeniden oder mit einem Komplexbildner ihres Kations assoziierten Alkalimetallhalogeniden oder Erdalkalimetallhalogeniden in einer Menge von 0,5 bis 10 mol-%, bezogen auf das alpha-chlorierte Carbonate, sowie bei einer Temperatur von 40 bis 150°C.

2. Verfahren nach Anspruch 1, dadurch gekennzeichnet, daß $R^1$ aliphatische $C_{1-8}$-Gruppe oder eine cycloaliphatische $C_{5-10}$-Gruppe darstellt.

3. Verfahren nach Anspruch 1 oder 2, dadurch gekennzeichnet, daß $R^2$ eine aliphatische $C_{1-8}$-Gruppe, eine cycloaliphatische $C_{5-10}$-Gruppe oder eine aromatischen $C_{6-10}$-Gruppe bedeutet, wobei der Substituent $R^2$ einen oder mehrere unter Aryl, Aryloxy, Alkoxy, Chlor, Fluor und Brom ausgewählte Substituenten aufweisen kann.

4. Verfahren nach einem der Ansprüche 1 bis 3, dadurch gekennzeichnet, daß die Verbindung ZBr in einer Menge von 1,05 bis 2 mol und vorzugsweise 1,1 bis 1,5 mol pro mol des alphachlorierten Carbonats eingesetzt wird.

5. Verfahren nach einem der Ansprüche 1 bis 4, dadurch gekennzeichnet, daß als Verbindung ZBr HBr,

$(CH_3)_3$—Si—Br oder 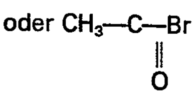

verwendet wird.

6. Verfahren nach einem der Ansprüche 1 bis 5, dadurch gekennzeichnet, daß der Katalysator in einer Menge von 1 bis 5 mol-%, bezogen auf das alpha-chlorierte Carbonat, eingesetzt wird.

7. Verfahren nach einem der Ansprüche 1 bis 6, dadurch gekennzeichnet, daß der Katalysator unter Pyridin, 4-Dimethylaminopyridin, quaternären Ammoniumchloriden und -bromiden, quaternären Phosphoniumchloriden und -bromiden, Hexabutylguanidiniumchlorid, mit 18-Krone-6 assoziiertem Kaliumchlorid und Magnesiumbromid ausgewählt wird.

8. Verfahren nach einem der Ansprüche 1 bis 7, dadurch gekennzeichnet, daß die Reaktion bei einer Temperatur von 60 bis 120°C durchgeführt wird.

9. Verfahren nach einem der Ansprüche 1 bis 8, dadurch gekennzeichnet, daß die Reaktion in Gegenwart eines Lösungsmittels durchgeführt wird.

10. Verfahren nach einem der Ansprüche 1 bis 9, dadurch gekennzeichnet, daß das Lösungsmittel unter den aromatischen und den aliphatischen Lösungsmitteln ausgewählt wird.

**Claims**

1. Process for the preparation of 1-bromoalkyl carbonates of formula

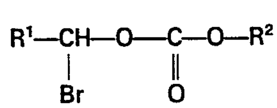

in which $R^1$ represents a straight-chain or branched $C_1$—$C_{20}$ aliphatic or $C_5$—$C_{15}$ alicyclic radical, and $R^2$ represents a $C_1$—$C_{12}$ aliphatic, $C_5$—$C_{20}$ alicyclic or $C_6$—$C_{14}$ aromatic radical, the radical $R^2$ being able to carry

one or more substituents chosen from the group comprising aryl, aryloxy and alkoxy radicals and chlorine, fluorine and bromine atoms, characterized in that an alpha-chlorinated carbonate of formula

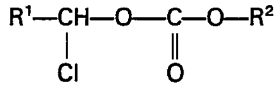

in which $R^1$ and $R^2$ have the above meanings is reacted with an anhydrous compound of formula ZBr in which Z represents a hydrogen atom, the group

$(CH_3)_3$—Si— or 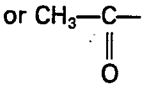

and, when $R^2$ is other than the aromatic radical, Z also represents a bromine atom, in the presence of a catalyst chosen from amongst heteroaromatic amines, quaternary ammonium or phosphonium halides, hexasubstituted guanidinium halides, alkali metal halides in combination with a complexing agent for their cation or alkaline earth metal halides, in a quantity of between 0.5 and 10 mole% relative to the alpha-chlorinated carbonate and at a temperature of between 40° and 150°C.

2. Process according to Claim 1, characterized in that $R^1$ represents a $C_1$—$C_8$ aliphatic or $C_5$—$C_{10}$ alicyclic radical.

3. Process according to Claim 1 or 2, characterized in that $R^2$ represents a $C_1$—$C_8$ aliphatic, $C_5$—$C_{10}$ alicyclic or $C_6$—$C_{10}$ aromatic radical, the radical $R^2$ being able to carry one or more substituents chosen from the group comprising aryl, aryloxy and alkoxy radicals and chlorine, fluorine and bromine atoms.

4. Process according to any one of the preceding claims, characterized in that the compound ZBr is employed in a quantity of between 1.05 and 2 moles per mole of alpha-chlorinated carbonate, preferably between 1.1 and 1.5.

5. Process according to any one of the preceding claims, characterized in that the compound ZBr employed is HBr,

$(CH_3)_3$—Si—Br or 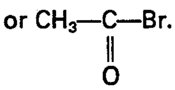

6. Process according to any one of the preceding claims, characterized in that the catalyst is employed in a quantity of between 1 and 5 mole% relative to the alpha-chlorinated carbonate.

7. Process according to any one of the preceding claims, characterized in that the catalyst employed is chosen from amongst pyridine, 4-dimethylaminopyridine, quaternary ammonium or phosphonium chlorides and bromides, hexabutylguanidinium chloride, potassium chloride in combination with 18-crown-6 and magnesium bromide.

8. Process according to any one of the preceding claims, characterized in that the reaction temperature is between 60° and 120°C.

9. Process according to any one of the preceding claims, characterized in that the reaction is carried out in the presence of a solvent.

10. Process according to the preceding claim, characterized in that the solvent is chosen from the group comprising aromatic solvents and aliphatic solvents.